# EUROPEAN PATENT APPLICATION

(11) **EP 4 000 620 A1**
(43) Date of publication of application: **25.05.2022**
(21) Application number: 20208998.3
(22) Date of filing: 20.11.2020
(51) Int. Cl.: A61K 31/45, A61K 31/4706, A61K 31/495, A61K 31/5375, A61K 31/54, A61K 31/573, A61P 31/12, A61P 31/14, A61P 31/16

(54) **LACTAM AND LACTONE DERIVATIVES FOR USE IN THE TREATMENT OF A VIRAL INFECTION SUCH AS COVID-19**

(71) Applicant: Université catholique de Louvain, 1348 Louvain-la-Neuve (BE)
(72) Inventor: SOUMILLION, Patrice, 1360 Perwez (BE); GALKA, Pierre, 59680 Colleret (FR)
(74) Representative: Icosa

(57) **Abstract**

The present invention relates to lactam or lactone derivatives of formula Ia: 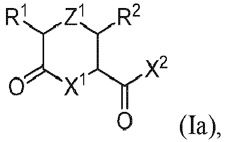 or a pharmaceutically acceptable salt, solvate or prodrug thereof, wherein X¹ is NH or O; X² is -OH, -O-alkyl or -NH-R⁵; Z¹ is S, SO, SO₂, O, CR³ or NR⁴; R¹, R², R³ and R⁴ each independently are H, alkyl or aryl; and R⁵ is H or a peptide group; and their use in treating a disease caused by a viral infection, especially a coronavirus infection, such as COVID-19 caused by SARS-CoV-2.

## Description

### FIELD OF INVENTION

The present invention relates to the treatment of a disease caused by a viral infection, especially a coronavirus infection, such as COVID-19. Especially, the present invention relates to lactam or lactone derivatives and their use in treating a disease caused by a viral infection, especially a coronavirus infection, such as COVID-19, in a subject in need thereof.

### BACKGROUND OF INVENTION

Coronaviruses (CoVs) are ribonucleic acid (RNA) viruses of the *Coronaviridae* family. Coronaviruses can cause a wide range of respiratory, gastrointestinal, neurologic, and systemic diseases. In most cases, human coronaviruses cause only mild respiratory infections, such as the common cold. However, in recent years, two highly pathogenic coronaviruses causing severe respiratory diseases emerged from animal reservoirs: severe acute respiratory syndrome coronavirus (SARS-CoV, also now referred to as SARS-CoV-1) first identified in 2003 and Middle East respiratory syndrome coronavirus (MERS-CoV) first identified in 2012.

In December 2019, the Wuhan Municipal Health Committee, China, identified a new infectious respiratory disease of unknown cause. Coronavirus RNA was quickly identified in some of the patients and in January 2020, a full genomic sequence of the newly identified human coronavirus SARS-CoV-2 (previously known as 2019-nCoV) was released by Shanghai Public Health Clinical Center & School of Public Health, Fudan University, Shanghai, China. The genomic sequence of SARS-COV-2 has 82% nucleotide identity with the genomic sequence of human SARS-CoV-1. Moreover, as previously shown for SARS-CoV-1, SARS-CoV-2 appears to utilize ACE2 (angiotensin converting enzyme 2) as receptor for viral cell entry (Hou et al., Cell, 2020, 182, 429-446).

In infected subjects exhibiting symptoms, the disease caused by SARS-COV-2 is now termed "coronavirus disease 2019" (COVID-19). COVID-19 is a respiratory illness with a broad clinical spectrum. The majority of affected subjects experience mild or moderate symptoms. COVID-19 generally presents first with symptoms including headache, muscle pain, fatigue, fever and respiratory symptoms (such as a dry cough, shortness of breath, and/or chest tightness). Other reported symptoms include a loss of smell (anosmia) and/or taste (ageusia). Some subjects develop a severe form of COVID-19 that may lead to pneumonitis and acute respiratory failure. Complications of COVID-19 also include thrombotic complications, pulmonary embolism, cardiovascular failure, renal failure, liver failure and secondary infections.

It is estimated that up to 25% of people around the world have at least one underlying condition that puts them at increased risk of severe COVID-19 if they are infected and that about 5% of the global population are likely to require hospitalization if infected. The disease severity is tightly associated with age and the presence of comorbidities.

Global efforts to identify an effective treatment for COVID-19 are ongoing. A number of clinical trials are thus currently underway to assess the efficacy of drugs. However, a therapeutic agent with a proven efficacy for preventing and/or treating COVID-19, in particular severe forms of COVID-19, is yet to be identified.

Therefore, there is still a need for effective treatment and prevention of diseases caused by a coronavirus, such as COVID-19, in particular to prevent the onset of severe forms of such diseases. There is also a need for effective treatment and prevention of diseases caused by other viruses.

The present invention relates to the use of lactam or lactone derivatives in the treatment of a disease caused by a viral infection, especially a coronavirus infection, such as COVID-19, in a subject in need thereof.

### SUMMARY

This invention thus relates to a compound of formula la, 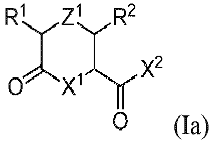 or a pharmaceutically acceptable salt, solvate or prodrug thereof, wherein
X¹ is NH or O;
X² is -OH, -O-alkyl or -NH-R⁵;
Z¹ is S, SO, SO₂, O, CR³ or NR⁴; and
R¹, R², R³ and R⁴ each independently are H, alkyl or aryl; and
R⁵ is H or a peptide group;
for use in the treatment of a disease caused by a viral infection in a subject in need thereof.

In one embodiment, the compound for use according to the invention is of formula la', as defined hereafter. In one embodiment, the compound for use according to the invention is of formula Ia-i or Ia-ii, as defined hereafter. In one embodiment, the compound for use according to the invention is of formula Ia-i' or Ia-ii', as defined hereafter. In one embodiment, the compound for use according to the invention is of formula Ia-i-1, Ia-i-2, Ia-i-3, Ia-i-4, Ia-i-5 or Ia-i-6, as defined hereafter. In one embodiment, the compound for use according to the invention is of formula Ia-i-1', Ia-i-2', Ia-i-3', Ia-i-4', Ia-i-5' or Ia-i-6', as defined hereafter.

In one embodiment, the compound for use according to the invention is selected from:
(R,S)-5-oxothiomorpholine-3-carboxylic acid;
(R)-5-oxothiomorpholine-3-carboxylic acid;
(R,S)-6-oxopiperidine-2-carboxylic acid;
(S)-6-oxopiperidine-2-carboxylic acid;
(R,S)-6-oxopiperazine-2-carboxylic acid;
(S)-6-oxopiperazine-2-carboxylic acid;
(R,S)-5-oxomorpholine-3-carboxylic acid;
(S)-5-oxomorpholine-3-carboxylic acid;
and pharmaceutically acceptable salts, solvates and prodrugs thereof.

In one embodiment, the viral infection is selected from coronavirus, rhinovirus, respiratory syncytial virus (RSV), seasonal influenza virus, human metapneumovirus, human parainfluenza viruses, highly pathogenic avian influenza A viruses (HPAIV), HIV, Ebola virus, measles virus, and yellow fever virus; preferably the viral infection is a coronavirus infection.

In one embodiment, the coronavirus is selected from HCoV-229E, HCoV-NL63, HCoV-OC43, HCoV-HKU1, MERS-CoV, SARS-CoV-1 and SARS-CoV-2; preferably the coronavirus is selected from MERS-CoV, SARS-CoV-1 and SARS-CoV-2; more preferably the coronavirus is SARS-CoV-2.

In one embodiment, the coronavirus is SARS-CoV-2 and the disease caused by the coronavirus infection is coronavirus disease 2019 (COVID-19).

In one embodiment, the subject is infected by the virus from less than 10 days, preferably from less than 6 days, more preferably from less than 4 days.

In one embodiment, the compound is to be administered simultaneously, separately or sequentially with at least one further pharmaceutically active agent selected from anti-viral agents, anti-interleukin 6 (anti-IL-6) agents, other agents such as chloroquine, hydroxychloroquine, or dexamethasone, and any mixtures thereof.

The invention also relates to a method for inhibiting a coronavirus 3C-like protease in a patient in need thereof, comprising administering to said patient an effective amount of a compound of formula Ia as herein defined.

The invention further relates to method for limiting the entry of a coronavirus in patient cells, comprising administering to patient in need thereof an effective amount of a compound of formula Ia as herein defined.

The invention also provides a method for lowering the viral load in a patient infected by a coronavirus, comprising administering to said patient an effective amount of a compound of formula Ia as herein defined.

### DEFINITIONS

In the present invention, the following terms have the following meanings:
- **"Alkyl"** refers to a hydrocarbyl radical of formula CₙH₂ₙ₊₁ wherein n is a number greater than or equal to 1. Generally, alkyl groups of this invention comprise from 1 to 6 carbon atoms, preferably from 1 to 4 carbon atoms, more preferably from 1 to 3 carbon atoms. Alkyl groups may be linear or branched. Suitable alkyl groups include methyl, ethyl, n-propyl, i-propyl, n-butyl, i-butyl, s-butyl and t-butyl, pentyl and its isomers (e.g. n-pentyl, iso-pentyl), and hexyl and its isomers (e.g. n-hexyl, iso-hexyl). Preferred alkyl groups include methyl, ethyl, n-propyl, i-propyl, n-butyl, i-butyl, s-butyl and t-butyl.
- **"Aryl"** refers to a polyunsaturated, aromatic hydrocarbyl group having a single ring (i.e. phenyl) or multiple aromatic rings fused together (e.g. naphthyl) or linked covalently, typically containing 5 to 12 carbon atoms; preferably 6 to 10 carbon atoms, wherein at least one ring is aromatic. The aromatic ring may optionally include one to two additional rings (either cycloalkyl, heterocyclyl or heteroaryl) fused thereto. Non-limiting examples of aryl comprise phenyl, biphenylyl, biphenylenyl, 5- or 6-tetralinyl, naphthalen-1- or -2-yl, 4-, 5-, 6 or 7-indenyl, 4- or 5-indanyl, 5-, 6-, 7- or 8-tetrahydronaphthyl, 1,2,3,4-tetrahydronaphthyl, 1,4-dihydronaphthyl.
- **"Disease caused by a coronavirus"** and **"disease caused by a coronavirus infection"** are interchangeable and refer to any symptom or set of symptoms induced in a subject by the presence of a coronavirus in the organism of said subject.
- **"Disease caused by a virus"** and **"disease caused by a viral infection"** are interchangeable and refer to any symptom or set of symptoms induced in a subject by the presence of a virus in the organism of said subject.
- **"Peptide group"** refers to a group comprised of amino acid residues covalently linked by peptide bonds. The amino acids are preferably of naturally occurring amino acids. The peptide groups of the invention include single amino acids. In the compounds of the invention, the peptide group contains from 1 to less than 50 amino acids; preferably, from 1 to 10 amino acids, more preferably from 1 to 5 amino acids. In the present invention, the peptide group comprises 1, 2, 3, 4 or 5 amino acids.
- **"Pharmaceutically acceptable excipient"** or **"pharmaceutically acceptable carrier"** refers to an excipient or carrier that does not produce an adverse, allergic or other untoward reaction when administered to a mammal, preferably a human. This includes any and all solvents, dispersion media, coatings, antibacterial and antifungal agents, isotonic agents, absorption delaying agents and other similar ingredients. For human administration, preparations should meet sterility, pyrogenicity, general safety and purity standards as required by the regulatory offices such as the FDA (US Food and Drug Administration) or EMA (European Medicines Agency).
- **"Respiratory support"** refers to any measure administered to a subject in order to compensate for a respiratory distress or failure experienced by the subject. Examples of such measures include non-invasive ventilation (NIV) such as supplemental oxygen (also called oxygen therapy) by mask or nasal prongs, positive pressure, high flow nasal oxygen; invasive mechanical ventilation (IMV) requiring tracheal intubation or tracheostomy; and extracorporeal membrane oxygenation (ECMO). As used herein, "respiratory support" or "oxygen therapy" thus encompasses both non-invasive ventilation (NIV) and invasive mechanical ventilation (IMV).
- **"Subject"** refers to a mammal, preferably a human. According to the present invention, a subject is a mammal, preferably a human, suffering from a disease caused by a virus, especially a coronavirus, in particular from COVID-19 caused by a SARS-CoV-2. In one embodiment, the subject is a **"patient",** *i.e.,* a mammal, preferably a human, who/which is awaiting the receipt of, or is receiving medical care or was/is/will be the object of a medical procedure, or is monitored for the development of a disease caused by a virus, especially a coronavirus, in particular COVID-19 caused by a SARS-CoV-2.
- **"Therapeutically effective amount"** or **"effective amount"** refers to the amount or dose or concentration of a compound according to the invention that is aimed at, without causing significant negative or adverse side effects to the subject in need of treatment, preventing, reducing, alleviating or slowing down (lessening) one or more of the symptoms or manifestations of a disease caused by a virus, especially a coronavirus, in particular COVID-19 caused by a SARS-CoV-2.
- **"Treating"** or **"Treatment"** refers to a therapeutic treatment, a prophylactic (or preventative) treatment, or both a therapeutic treatment and a prophylactic (or preventative) treatment, wherein the object is to prevent, reduce, alleviate, and/or slow down (lessen) one or more of the symptoms or manifestations of a disease caused by a virus, especially a coronavirus, in particular COVID-19 caused by a SARS-CoV-2, in a subject in need thereof. Symptoms of a disease caused by a virus, especially a coronavirus, in particular COVID-19 caused by a SARS-CoV-2, include, without being limited to, headache, muscle pain, fatigue, fever, anosmia, ageusia, and respiratory symptoms such as dry cough and/or breathing difficulties that may require respiratory support (for example supplemental oxygen, non-invasive ventilation, invasive mechanical ventilation, extracorporeal membrane oxygenation (ECMO)). Manifestations of a disease caused by a virus, especially a coronavirus, in particular COVID-19 caused by a SARS-CoV-2, also include, without being limited to, the viral load (also known as viral burden or viral titer) detected in a biological sample from the subject. In one embodiment, "treating" or "treatment" refers to a therapeutic treatment. In another embodiment, "treating" or "treatment" refers to a prophylactic or preventive treatment. In yet another embodiment, "treating" or "treatment" refers to both a prophylactic (or preventive) treatment and a therapeutic treatment. In one embodiment, the object of the treatment according to the present invention is to bring about at least one of the following:
   ∘ an improvement in the clinical status, for example defined as a decrease in the score assessed according with an ordinal scale such as the 8-point ordinal scale as defined hereinafter;
   ∘ a decrease in the requirement for respiratory support;
   ∘ a decrease in the requirement for other organ support, such as cardiovascular support and/or renal replacement therapy;
   ∘ a discharge from the intensive care unit;
   ∘ a discharge from hospital; and/or
   ∘ a reduction in the viral load detected in a sample from the subject.
- **"8-point ordinal scale"** as used herein refers to a tool for assessing the clinical status of a subject suffering from a disease caused by a virus, especially a coronavirus, in particular COVID-19 caused by a SARS-CoV-2. The 8-point ordinal scale ranges from 1 to 8, with a lower score corresponding to a better clinical status as indicated below:
   ∘ a score of 1 corresponds to a subject not hospitalized, with no limitations on activities;
   ∘ a score of 2 corresponds to a subject not hospitalized, with limitations on activities;
   ∘ a score of 3 corresponds to a subject hospitalized, not requiring supplemental oxygen;
   ∘ a score of 4 corresponds to a subject hospitalized, requiring supplemental oxygen by mask or nasal prongs;
   ∘ a score of 5 corresponds to a subject hospitalized, on non-invasive ventilation or high flow oxygen devices;
   ∘ a score of 6 corresponds to a subject hospitalized, on intubation and mechanical ventilation;
   ∘ a score of 7 corresponds to a subject hospitalized, on invasive mechanical ventilation and additional organ support such as pressors, renal replacement therapy (RRT) and extracorporeal membrane oxygenation (ECMO);
   ∘ a score of 8 corresponds to death.

### DETAILED DESCRIPTION

The present invention thus relates to the treatment of a disease caused by a viral infection, especially a coronavirus infection, such as COVID-19 caused by SARS-CoV-2, in a subject in need thereof, using lactam or lactone compounds.

### Compounds

The compounds of the invention are lactam and lactone derivatives, preferably of general formula I: 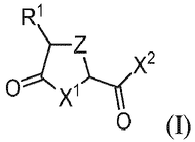 or a pharmaceutically acceptable salt, solvate or prodrug thereof, wherein
X¹ is NH or O;
X² is -OH, -O-alkyl or -NH-R⁵;
Z is -CR²- or -Z¹-CR²-;
Z¹ is S, SO, SO₂, O, CR³ or NR⁴;
R¹, R², R³ and R⁴ each independently are H, alkyl or aryl; and
R⁵ is H or a peptide group.

In one embodiment, the compound is of formula I': 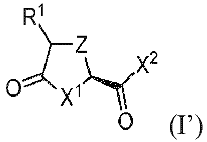 or a pharmaceutically acceptable salt, solvate or prodrug thereof, wherein X¹, X², Z and R¹ are as defined above.

In one embodiment, the compound is of formula Ia: 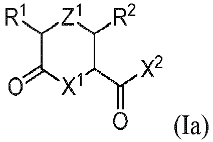 or a pharmaceutically acceptable salt, solvate or prodrug thereof, wherein X¹, X², Z¹, R¹ and R² are as defined above.

In one embodiment, the compound is of formula Ia': 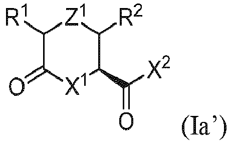 or a pharmaceutically acceptable salt, solvate or prodrug thereof, wherein X¹, X², Z¹, R¹ and R² are as defined above.

In one embodiment, the compound is of formula Ia-i or Ia-ii: 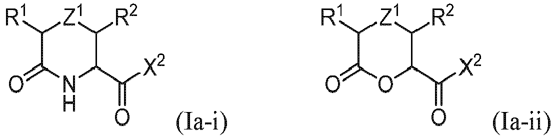 or a pharmaceutically acceptable salt, solvate or prodrug thereof, wherein X², Z¹, R¹ and R² are as defined above.

In one embodiment, the compound is of formula Ia-i' or Ia-ii': 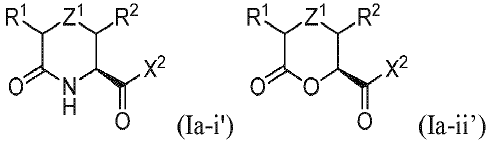 or a pharmaceutically acceptable salt, solvate or prodrug thereof, wherein X², Z¹, R¹ and R² are as defined above.

In one embodiment, the compound is of formula Ia-i-1, Ia-i-2, Ia-i-3, Ia-i-4, Ia-i-5 or Ia-i-6: 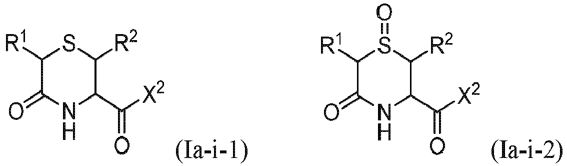 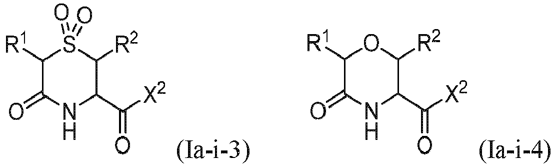 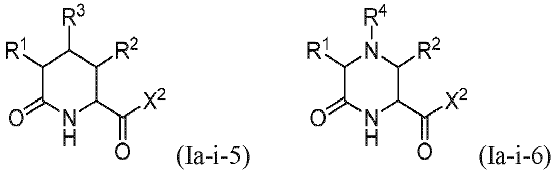 or a pharmaceutically acceptable salt, solvate or prodrug thereof, wherein X², R¹, R², R³ and R⁴ are as defined above.

In one embodiment, the compound is of formula Ia-i-1', Ia-i-2', Ia-i-3', Ia-i-4', Ia-i-5' or Ia-i-6' : 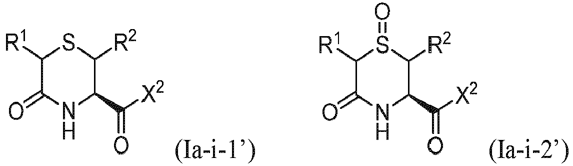 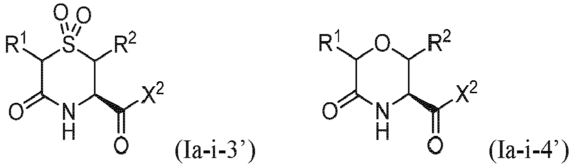 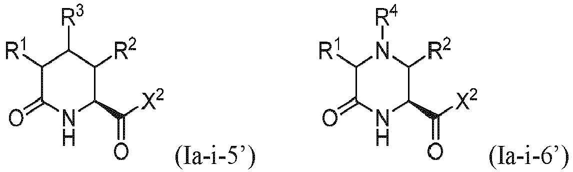 or a pharmaceutically acceptable salt, solvate or prodrug thereof, wherein X², R¹, R², R³ and R⁴ are as defined above.

In one embodiment, the compound is of formula Ib: 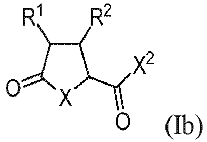 or a pharmaceutically acceptable salt, solvate or prodrug thereof, wherein X¹, X², R¹ and R² are as defined above.

In one embodiment, the compound is of formula Ib': 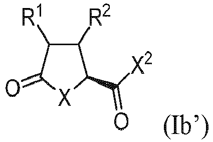 or a pharmaceutically acceptable salt, solvate or prodrug thereof, wherein X¹, X², R¹ and R² are as defined above.

In one embodiment, the compound is of formula Ib-i or Ib-ii: 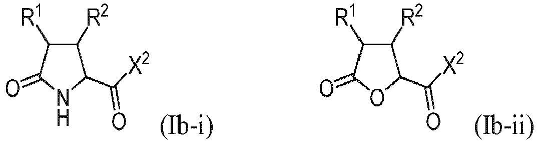 or a pharmaceutically acceptable salt, solvate or prodrug thereof, wherein X², R¹ and R² are as defined above.

In one embodiment, wherein the compound is of formula Ib-i' or Ib-ii': 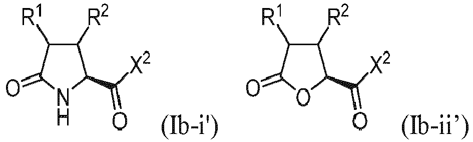 or a pharmaceutically acceptable salt, solvate or prodrug thereof, wherein X², R¹ and R² are as defined above.

In one embodiment, in above formulae, X² is -OH or -O-alkyl, preferably -OH. In another embodiment, in above formulae, X² is -NH-R⁵ wherein R⁵ is H or a peptide group.

In one embodiment, when Z is -CR²-, at least one of R¹ and R² is not a hydrogen.

In one embodiment, the compound is selected from:
(R,S)-5-oxothiomorpholine-3-carboxylic acid;
(R)-5-oxothiomorpholine-3-carboxylic acid (lactam 2);
(R,S)-6-oxopiperidine-2-carboxylic acid;
(S)-6-oxopiperidine-2-carboxylic acid (lactam 3);
(R,S)-6-oxopiperazine-2-carboxylic acid;
(S)-6-oxopiperazine-2-carboxylic acid;
(R,S)-5-oxomorpholine-3-carboxylic acid;
(S)-5-oxomorpholine-3-carboxylic acid;
and pharmaceutically acceptable salts, solvates and prodrugs thereof.

The compounds of formula I and subformulae thereof contain at least one asymmetric centre and thus may exist as different stereoisomeric forms. Accordingly, all references to compounds of formula I include references to all possible stereoisomers and includes not only the racemic compounds but the individual enantiomers and their non-racemic mixtures as well. When a compound is desired as a single enantiomer, such single enantiomer may be obtained by stereospecific synthesis, by resolution of the final product or any convenient intermediate, or by chiral chromatographic methods as each are known in the art. Resolution of the final product, an intermediate, or a starting material may be carried out by any suitable method known in the art.

Bonds from an asymmetric carbon in compounds are generally depicted using a solid line ( 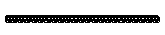 ), a solid wedge ( 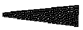 ), or a dotted wedge ( 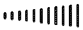 ).The use of either a solid or dotted wedge to depict bonds from an asymmetric carbon atom is meant to indicate that only the stereoisomer shown is meant to be included.

All references to compounds of the invention include references to isotopically-labelled compounds of the invention, including deuterated compounds.

All references to compounds of the invention include references to salts, solvates, multi-component complexes and liquid crystals thereof. All references to compounds of the invention include references to polymorphs and crystal habits thereof. All references to compounds of the invention include references to pharmaceutically acceptable prodrugs thereof.

The compounds of the invention may be in the form of pharmaceutically acceptable salts. Pharmaceutically acceptable salts of the compounds of formula I include the acid addition and base salts thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminum, ammonium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.

Hemisalts of acids and bases may also be formed, for example, hemisulphate and hemicalcium salts.

When the compounds of formula I contain an acidic group as well as a basic group the compounds of the invention may also form internal salts, and such compounds are within the scope of the invention. When the compounds of the invention contain a hydrogen-donating heteroatom (e.g., NH), the invention also covers salts and/or isomers formed by transfer of said hydrogen atom to a basic group or atom within the molecule.

Pharmaceutically acceptable salts of compounds of formula I may be prepared by one or more of these methods:
(i) by reacting the compound of formula I with the desired acid;
(ii) by reacting the compound of formula I with the desired base;
(iii) by removing an acid- or base-labile protecting group from a suitable precursor of the compound of formula I or by ring-opening a suitable cyclic precursor, e.g., a lactone or lactam, using the desired acid; and/or
(iv) by converting one salt of the compound of formula I to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

Although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of Formula I above.

The compounds of the invention may be in the form of pharmaceutically acceptable solvates. The term **"solvate"** is used herein to describe a molecular complex comprising a compound of the invention and contains stoichiometric or sub-stoichiometric amounts of one or more pharmaceutically acceptable solvent molecule such as ethanol. The term **"hydrate"** refers to a solvate when said solvent is water.

The compounds of the invention may be in the form of pharmaceutically acceptable prodrugs. The term **"prodrug"** as used herein means the pharmacologically acceptable derivatives of compounds of formula I, such as esters, whose in vivo biotransformation product is the active drug. Prodrugs are characterized by increased bio-availability and are readily metabolized into the active compounds in vivo.

Suitable prodrugs for the purpose of the invention include carboxylic esters, in particular alkyl esters, aryl esters, acyloxyalkyl esters, and dioxolene carboxylic esters; ascorbic acid esters.

Suitable prodrugs for the purpose of the invention also include non-cyclized derivatives of the lactam and lactone compounds of the invention that can undergo condensation in vivo. Such condensation may be catalysed by endogenous enzymes such as glutaminyl cyclase. For example, a prodrug of 5-oxothiomorpholine-3-carboxylic acid can be carboxymethyl-cysteine:

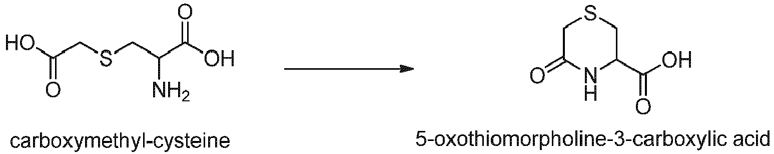

The compounds of the invention can be manufactured according to methods known by one skilled in the art.

### Disease

This invention thus relates to compounds for use in the treatment of a disease caused by a viral infection.

In one embodiment, the virus is causing syncytia, such as for example pneumoviruses (e.g. respiratory syncytial virus (RSV)), coronavirus (e.g. COVID-19).

In one embodiment, the virus is selected from coronavirus, rhinovirus, respiratory syncytial virus (RSV), seasonal influenza virus, human metapneumovirus, human parainfluenza viruses, highly pathogenic avian influenza A viruses (HPAIV), HIV, Ebola virus, measles virus, and yellow fever virus. In a preferred embodiment, the virus is a coronavirus.

This invention thus especially relates to compounds for use in the treatment of a disease caused by a coronavirus infection. In one embodiment, the coronavirus is a human coronavirus. In one embodiment, the coronavirus is an alpha coronavirus or a beta coronavirus, preferably a beta coronavirus.

Examples of alpha coronaviruses include, without being limited to, human coronavirus 229E (HCoV-229E) and human coronavirus NL63 (HCoV-NL63) also sometimes known as HCoV-NH or New Haven human coronavirus.

Examples of beta coronaviruses include, without being limited to, human coronavirus OC43 (HCoV-OC43), human coronavirus HKU1 (HCoV-HKU1), Middle East respiratory syndrome-related coronavirus (MERS-CoV) previously known as novel coronavirus 2012 or HCoV-EMC, severe acute respiratory syndrome coronavirus (SARS-CoV) also known as SARS-CoV-1 or SARS-classic, and severe acute respiratory syndrome coronavirus (SARS-CoV-2) also known as 2019-nCoV or novel coronavirus 2019.

In one embodiment, the coronavirus is selected from the group comprising or consisting of HCoV-229E, HCoV-NL63, HCoV-OC43, HCoV-HKU1, MERS-CoV, SARS-CoV-1 and SARS-CoV-2. In one embodiment, the coronavirus is selected from the group comprising or consisting of MERS-CoV, SARS-CoV-1 and SARS-CoV-2.

In one embodiment, the coronavirus is a MERS coronavirus, in particular MERS-CoV causing Middle East respiratory syndrome (MERS). Thus, in one embodiment, the subject is suffering from MERS caused by MERS-CoV.

In one embodiment, the coronavirus is a SARS coronavirus. In one embodiment, the coronavirus is SARS-CoV (also referred to as SARS-CoV-1) causing severe acute respiratory syndrome (SARS) or SARS-CoV-2 causing COVID-19. Thus, in one embodiment, the subject is suffering from SARS caused by SARS-CoV-1 or from COVID-19 caused by SARS-CoV-2. In one embodiment, the coronavirus is SARS-CoV-2 causing COVID-19. Thus, in one in one embodiment, the subject is suffering from COVID-19 caused by SARS-CoV-2.

Thus, in one embodiment, the compounds of the invention are for use in the treatment of a coronavirus infection selected from MERS-CoV, SARS-CoV-1 and SARS-CoV-2. In one embodiment, the compounds of the invention are for use in the treatment of MERS, SARS and COVID-19. According to a preferred embodiment, the compounds of the invention are for use in the treatment of COVID-19.

As mentioned above "disease caused by a virus" or "disease caused by a viral infection" are interchangeable and refer to any symptom or set of symptoms induced in a subject by the presence of a virus in the organism of said subject.

Diseases caused by a virus, especially a coronavirus such as COVID-19 caused by a SARS-CoV-2, have a broad clinical spectrum, from mild or moderate symptoms to more severe forms, with a wide variety of complications.

Especially, subjects affected by COVID-19 generally present first with symptoms including headache, muscle pain, fatigue, fever and respiratory symptoms such as dry cough and/or breathing difficulties that may require respiratory support (for example supplemental oxygen, non-invasive ventilation, invasive mechanical ventilation, extracorporeal membrane oxygenation (ECMO)). Other reported symptoms include anosmia and/or ageusia.

Some subjects develop a severe form of COVID-19 that may lead to respiratory complications such as pneumonitis and acute respiratory failure. Complications of COVID-19 also include extra-respiratory complications such as thrombotic complications, pulmonary embolism, cardiovascular failure, renal failure, liver failure and secondary infections.

In one embodiment, the severity of the disease caused by a virus, especially a coronavirus such as COVID-19 caused by a SARS-CoV-2, can be evaluated by the World Health Organization (WHO) COVID ordinal scale for clinical improvement. This WHO COVID ordinal scale provides a score ranging from 0 to 8 depending on the patient's state, as shown in Table 1 hereafter.

**Table 1: WHO COVID ordinal scale**

| **Patient State** | **Descriptor** | **Score** |
|---|---|---|
| Uninfected | No clinical or virological evidence of infection | 0 |
| Ambulatory Mild disease | No limitation of activities | 1 |
| | Limitation of activities | 2 |
| Hospitalized Moderate disease | Hospitalized, no oxygen therapy | 3 |
| | Oxygen by mask or nasal prongs | 4 |
| Hospitalized Severe disease | Non-invasive ventilation or high-flow oxygen | 5 |
| | Intubation and mechanical ventilation | 6 |
| | Ventilation + additional organ support - pressors, renal replacement therapy (RRT), extracorporeal membrane oxygenation (ECMO) | 7 |
| Dead | Death | 8 |

In one embodiment, a mild form of COVID-19 corresponds to a score of 1 or 2 according to the WHO COVID ordinal scale. In one embodiment, a moderate form of COVID-19 corresponds to a score of 3 or 4 according to the WHO COVID ordinal scale. In one embodiment, a severe form of COVID-19 corresponds to a score ranging from 5 to 7 according to the WHO COVID ordinal scale.

In one embodiment, the severity of COVID-19 caused by a SARS-CoV-2 can also be evaluated according to the WHO criteria of severity as follows:
- mild: cases showing mild clinical symptoms without evidence of viral pneumonia or hypoxia.
- moderate: cases showing fever and respiratory symptoms (such as a cough, shortness of breath, and/or chest tightness) with radiological findings of pneumonia and that may require (O₂): 3L/min < oxygen < 5L/min
- severe: cases meeting any of the following criteria:
   - respiratory distress (respiratory rate (RR) ≧ 30 breaths/ min);
   - oxygen saturation (SpO₂) ≤ 93% at rest in ambient air; or SpO₂ ≤ 97% with O₂ > 5L/min;
   - ratio of artery partial pressure of oxygen/inspired oxygen fraction (PaO₂/FiO₂) ≦ 300 mmHg (1 mmHg = 0.133 kPa), PaO₂/FiO₂ in high-altitude areas (at an altitude of over 1,000 meters above the sea level) shall be corrected by the following formula: PaO₂/FiO₂ [multiplied by] [Atmospheric pressure (mmHg)/760]; and/or
   - chest imaging that showed obvious lesion progression within 24-48 hours > 50%.
- critical: cases meeting any of the following criteria:
   - respiratory failure and requiring mechanical ventilation;
   - shock; and/or
   - multiple organ failure (extra pulmonary organ failure) requiring admission to intensive care unit (ICU).

In one embodiment, a severe form of the disease caused by a coronavirus, in particular a severe form COVID-19, is defined as requiring hospitalization. In one embodiment, a severe form of the disease caused by a coronavirus, in particular a severe form COVID-19, is defined as requiring admission in intensive care unit (ICU).

In one embodiment, a severe form of the disease caused by a coronavirus, in particular a severe form COVID-19, is defined as requiring respiratory support as defined hereinabove. In one embodiment, the respiratory support is selected from the group comprising or consisting of non-invasive ventilation (NIV) such as supplemental oxygen (also called oxygen therapy) by mask or nasal prongs, positive pressure, high flow nasal oxygen; invasive mechanical ventilation (IMV) requiring tracheal intubation or tracheostomy; and extracorporeal membrane oxygenation (ECMO). In one embodiment, a severe form of the disease caused by a coronavirus, in particular a severe form COVID-19, is defined as requiring invasive mechanical ventilation as described hereinabove.

In one embodiment, the complication of the disease caused by a coronavirus, in particular of COVID-19, is selected from the group comprising or consisting of, respiratory failure, including acute respiratory failure or acute respiratory distress syndrome (ARDS); persistence of respiratory failure including the requirement for prolonged mechanical ventilation, in particular prolonged mechanical ventilation lasting more than 15 days, and failed extubation; secondary infection or superinfection; thrombotic complications including venous and/or arterial thromboembolism; pulmonary embolism; cardiocirculatory failure (which may also be referred to as cardiovascular failure); renal failure including acute kidney injury (AKI); liver failure; and any combinations thereof.

### Subject

In one embodiment, the subject is a male. In one embodiment, the subject is a female.

In a preferred embodiment, the subject is an adult. Thus, in one embodiment, the subject is older than 18 years of age. In one embodiment, the subject is older than 60, 65, 70, 75, 80 or 85 years of age. In one embodiment, the subject is younger than 85, 80, 75, 70, 65 or 60 years of age. In another embodiment the subject is a child. Thus, in one embodiment, the subject is younger than 18, 17, 16 or 15 years of age.

In one embodiment, the subject is diagnosed with a SARS-CoV-2 infection. Methods for diagnosing a SARS-CoV-2 infection include, but are not limited to, rRT-PCR (real-time reverse transcription polymerase chain reaction) test allowing to detect the presence of SARS-CoV-2 in a sample from a subject (such as a sample from a nasal swab, a sample from an oropharyngeal swab, a sputum sample, a lower respiratory tract aspirate, a bronchoalveolar lavage, a nasopharyngeal wash/aspirate or a nasal aspirate) or an antibody test (such as an enzyme-linked immunosorbent assay (ELISA)) allowing to detect the presence of antibodies against SARS-CoV-2 in a sample from a subject (such as a blood sample).

In a specific embodiment, the subject is infected by SARS-CoV-2 from less than 10 days, preferably from less than 6 days, more preferably from less than 4 days. The compounds of the invention are particularly useful for subjects being at a very early stage of the infection by the virus.

In another embodiment, the subject is infected by SARS-CoV-2 from more than 10 days.

In one embodiment, the subject presents at least one of the following symptoms: cough, shortness of breath or difficulty breathing. In one embodiment, the subject presents at least one, preferably at least two, of the following symptoms: fever (*i*.*e*., any body temperature over 38°C), chills, repeated shaking with chills, muscle pain, headache, sore throat and new loss of taste or smell.

In one embodiment, the subject is suffering from a mild form of COVID-19, corresponding to a score of 1 or 2 according to the WHO COVID scoring scale (according to Table 1 above). In one embodiment, the subject is suffering from a moderate form of COVID-19, corresponding to a score of 3 or 4 according to the WHO COVID scoring scale. In another embodiment, the subject is suffering from a severe form of COVID-19 corresponding to a score ranging from 5 to 7 according to the WHO COVID scoring scale.

In one embodiment, the subject is not hospitalized. In one embodiment, the subject is hospitalized.

In one embodiment, the subject is hospitalized but does not require admission in ICU. In one embodiment, the subject is hospitalized and requires admission in ICU. In one embodiment, the subject is hospitalized in ICU.

In one embodiment, the subject is hospitalized and does not require respiratory support.

In one embodiment, the subject is hospitalized and requires respiratory support. In one embodiment, the subject is hospitalized and requires non-invasive ventilation (NIV). In one embodiment, the subject is hospitalized in ICU and requires invasive mechanical ventilation. In one embodiment, the subject is hospitalized in ICU and is under invasive mechanical ventilation. In one embodiment, the subject is hospitalized in ICU and has been under invasive mechanical ventilation for less than 6 hours, 12 hours, 18 hours, 24 hours, 30 hours, 36 hours, 42 hours, or 48 hours, preferably for less than 48 hours.

In one embodiment, the subject does not suffer from any underlying condition or disease.

In another embodiment, the subject suffers from at least one comorbidity. As used herein, "comorbidity" refers to a disease or condition coexisting in the subject with the disease caused by a virus, especially a coronavirus.

Examples of comorbidities that may coexist in the subject with a disease caused by a virus, especially a coronavirus such as a SARS-CoV-2 infection causing COVID-19, include, without being limited to, acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), cystic fibrosis, diabetes, emphysema, hematological diseases, high blood pressure, immunodeficiency, infection with HIV, malignancy (*i*.*e*., cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions.

In one embodiment, the subject presents at least one comorbidity selected from the group comprising or consisting of acute kidney injury, asthma, atopy, autoimmune or auto-inflammatory diseases or conditions, bone marrow or stem cell transplantations in the past 6 months, bronchial hyperreactivity, cardiovascular diseases or conditions, chronic bronchitis, chronic kidney diseases, chronic liver disease, chronic obstructive pulmonary disease (COPD), ciliary deficiencies (hereditary or acute ciliary deficiencies), cystic fibrosis, diabetes, emphysema, hematological diseases, high blood pressure, immunodeficiency, infection with HIV, malignancy (*i*.*e*., cancer), obesity, pulmonary hypertension, rare diseases and inborn errors of metabolism that significantly increase the risk of infections (such as severe combined immunodeficiency), reactive airway disease, recipient of solid organ transplants, and severe respiratory conditions.

### Therapeutic effect

The compounds of the invention as described above are thus useful for the treatment and/or prevention of a disease caused by a viral infection, especially a coronavirus infection in a subject in need thereof, especially for the treatment of COVID-19 caused by a SARS-CoV-2 infection in a subject in need thereof. The compounds of the invention are especially very useful at the early stages of the infection by the virus to prevent the development of the disease. The compounds of the invention may also be useful at a more advanced stage of the infection.

In one embodiment, the compounds of the invention are inhibitors of coronavirus main protease (Mpro) also referred to as 3C-like protease (3CLpro), preferably compounds of the invention are inhibitors of SARS-CoV-2 Mpro.

The Mpro is crucial for virus replication and controlling the host cell response. Therefore, it is a key target for antiviral drugs. Indeed, the Mpro plays an essential role in coronavirus replication by digesting the viral polyproteins at several sites. The Mpro has a unique substrate preference for glutamine at the P1 site, a feature that is absent in closely related host proteases, enabling to achieve high selectivity by targeting viral Mpro.

The term **"inhibitor"** refers to a compound that has a biological effect to inhibit or significantly reduce or down-regulate the expression of a gene and/or a protein or that has a biological effect to inhibit or significantly reduce the biological activity of a protein. Consequently, a **"3CLpro inhibitor"** or a **"Mpro inhibitor",** which are interchangeable terms, refers to a compound that has a biological effect to inhibit or significantly reduce or down-regulate the expression of the gene encoding for 3CLpro and/or the expression of 3CLpro and/or the biological activity of 3CLpro.

The invention thus also relates to a method for inhibiting a coronavirus 3C-like protease in a patient in need thereof, comprising administering to said patient an effective amount of a compound according to the invention.

In one embodiment, the compounds of the invention are effective in preventing the entry of the virus into host cells. By limiting this critical stage of the life of the virus, it enables to avoid viral replication. Without willing to be bound by a theory, the compounds of the invention might be inhibitors of host proteases that contribute to virus entry into host cells by processing the spike protein of the virus. Especially, the compounds of the invention might be inhibitors of furin and/or transmembrane serine protease 2 (TMPRSS2) host proteases. TMPRSS2 was shown to activate the fusion proteins of a number of respiratory viruses, including human metapneumovirus, human parainfluenza viruses, and coronaviruses, including SARS-CoV-2, SARS-CoV and Middle East respiratory syndrome (MERS)-CoV in vitro. Furin has been identified as an activating protease for the fusion proteins of a broad range of viruses, including SARS-CoV-2, highly pathogenic avian influenza A viruses (HPAIV), HIV, Ebola virus, measles virus, and yellow fever virus.

The invention thus also relates to a method for limiting the entry of a virus, especially a coronavirus in patient cells, comprising administering to patient in need thereof an effective amount of a compound according to the invention.

The invention also relates to a method for inhibiting TMPRSS2 and/or furin protease in a patient in need thereof, comprising administering to said patient an effective amount of a compound according to the invention.

The invention thus also relates to a method for lowering the viral load in a patient infected by a virus, especially a coronavirus, comprising administering to said patient an effective amount of a compound according to the invention.

Above methods are especially useful when the coronavirus is selected from HCoV-229E, HCoV-NL63, HCoV-OC43, HCoV-HKU1, MERS-CoV, SARS-CoV-1 and SARS-CoV-2; preferably the coronavirus is selected from MERS-CoV, SARS-CoV-1 and SARS-CoV-2; more preferably the coronavirus is SARS-CoV-2.

### Combination with other active agents

In one embodiment, the compound according to the invention is administrated to the subject as sole therapeutic agent.

In another embodiment, the compound according to the invention is administrated to the subject in combination with at least one further pharmaceutically active agent.

In one embodiment, the compound of the invention is to be administered simultaneously, separately or sequentially with at least one further pharmaceutically active agent.

Examples of further pharmaceutically active agents include, but are not limited to, anti-viral agents, anti-interleukin 6 (anti-IL-6) agents and other agents such as chloroquine, hydroxychloroquine, or dexamethasone.

In one embodiment, the at least one further pharmaceutically active agent is an antiviral agent, an anti-IL-6 agent, chloroquine, hydroxychloroquine, or any mixtures thereof.

Example of antiviral agents include, without being limited to, remdesivir, and a combination of lopinavir and ritonavir (lopinavir/ritonavir).

In one embodiment, the at least one further pharmaceutically active agent is remdesivir, or a combination of lopinavir and ritonavir (lopinavir/ritonavir).

As used herein, anti-IL-6 agents target either IL-6 (interleukin 6 or interleukin-6) or its receptor (IL-6R). Example of anti-IL-6 agents include, without being limited to, tocilizumab and sarilumab.

In one embodiment, the at least one further pharmaceutically active agent is tocilizumab or sarilumab.

In one embodiment, the at least one further pharmaceutically active agent is selected from the group comprising or consisting of remdesivir, a combination of lopinavir and ritonavir, tocilizumab, sarilumab, chloroquine, hydroxychloroquine, dexamethasone and any mixtures thereof. In one embodiment, the at least one further pharmaceutically active agent is chloroquine or hydroxychloroquine, preferably hydroxychloroquine.

In one embodiment, the subject receives a compound according to the invention as part of a treatment protocol. In one embodiment, said treatment protocol further comprises, before, concomitantly or after the administration of the compound according to the invention, the administration of another pharmaceutically active agent, as described herein. Therefore, the subject to be treated was previously treated or is to be treated with another pharmaceutically active agent.

Another object of the invention is thus a kit-of-part comprising, in a first part, at least one compound according to the invention and, in a second part, another pharmaceutically active agent, as described hereinabove.

### Administration

This invention also relates to a pharmaceutical composition comprising a compound according to the invention, as described hereinabove, and at least one pharmaceutically acceptable carrier.

According to one embodiment, the compounds according to the invention may be administered by oral, parenteral (e.g., intramuscular, intraperitoneal, intravenous, ICV, intracisternal injection or infusion, subcutaneous injection, or implant), by inhalation spray, nasal, rectal, sublingual, or topical routes of administration and may be formulated in suitable dosage unit formulations containing conventional non-toxic pharmaceutically acceptable carriers, adjuvants and vehicles appropriate for each route of administration.

The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active ingredient is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

In one embodiment, the compound according to the invention is administered by oral route. In one embodiment, the compound according to the invention is formulated as a pharmaceutical composition containing the compound according to the invention in a form suitable for oral use, for example, as tablets, troches, lozenges, aqueous or oily suspensions, dispersible powders or granules, emulsions, hard or soft capsules, or syrups or elixirs.

In one embodiment, the compound according to the invention is administered by injection. In one embodiment, the compound according to the invention is formulated as a pharmaceutical composition containing the compound according to the invention in a form suitable for injection. In one embodiment, the compound according to the invention is administered by infusion, preferably by intravenous infusion. In another embodiment, the compound according to the invention is injected intraperitoneally. In another embodiment, the compound according to the invention is injected intradermally.

The pharmaceutical compositions may be in the form of a sterile injectable aqueous or oleaginous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables.

In one embodiment, the pharmaceutical composition comprises the compound according to the invention as sole therapeutic agent. In another embodiment, the pharmaceutical composition further comprises at least one other therapeutic agent, as those described above.

This invention thus relates to the use of lactam or lactone derivatives, preferably of formula I or subformulae thereof as described above in the treatment of a disease caused by a viral infection, especially a coronavirus infection, as described hereinabove.

The present invention also provides a pharmaceutical composition comprising a lactam or lactone derivative, preferably of formula I or subformulae thereof as described above, and a pharmaceutically acceptable carrier, for use in the treatment of a disease caused by a viral infection, especially a coronavirus infection.

The present invention further relates to the use of a lactam or lactone derivative, preferably of formula I or subformulae thereof as described above, for the manufacture of a medicament for treating a disease caused by a viral infection, especially a coronavirus infection.

The present invention also relates to a method for treating a disease caused by a viral infection, especially a coronavirus infection, comprising administering to a subject in need thereof a therapeutically effective amount of a lactam or lactone derivative, preferably of formula I or subformulae thereof as described above.

### BRIEF DESCRIPTION OF THE DRAWINGS

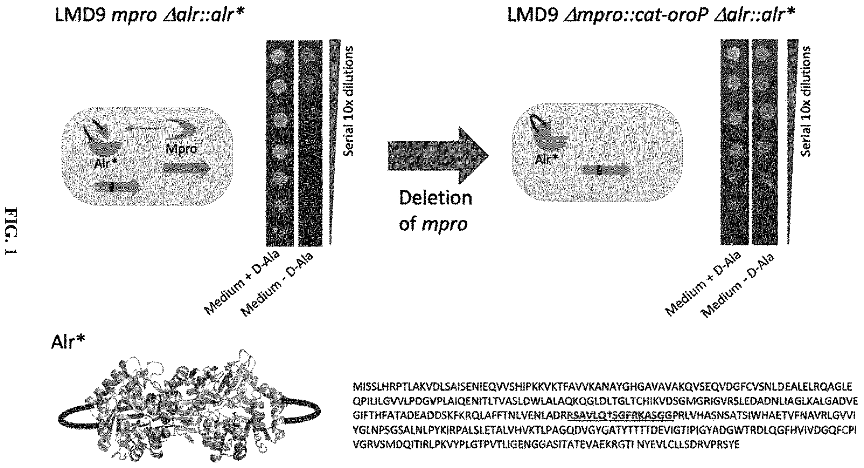

**Figure 1****:** Top left: LMD9m (LMD9 *mpro* Δ*alr::alr**) produces the SARS-CoV-2 Mpro protease and an engineered alanine racemase (Alr*) has been engineered by inserting a Mpro cleavage site in a surface loop. By cleaving Alr*, Mpro impairs the growth of the strain in a medium without D-Alanine. Top right: deleting the mpro gene from LMD9m is reverting the phenotype demonstrating the role of the protease. Bottom left: based on the structure of Air from Streptococcus pneumoniae (homologous enzyme, pdb code 3s46), the site for inserting the Mpro cleavage site was chosen (dark loops). Bottom right: primary sequence of Alr* with Mpro cleavage site (underlined) inserted between R194 and Pro195. The cross indicates the precise site of cleavage.

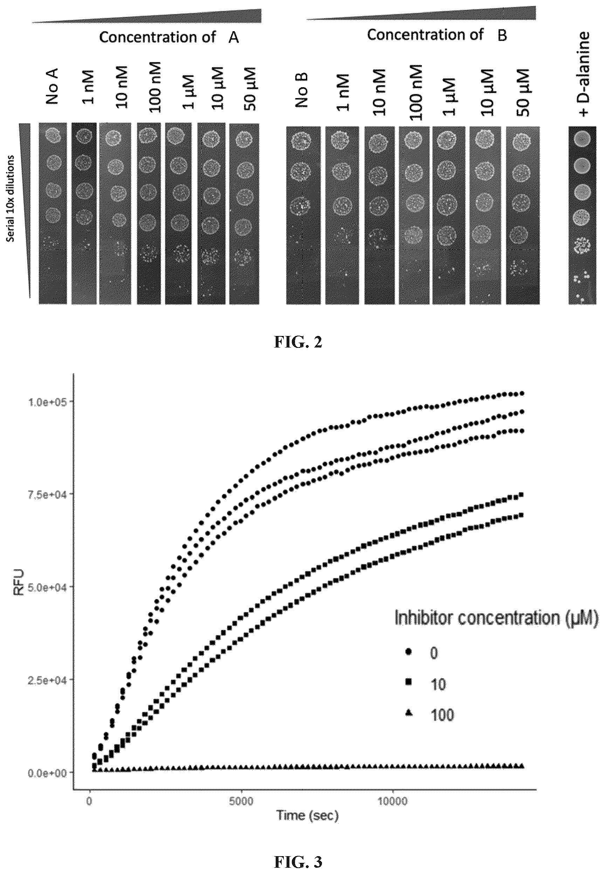

**Figure 2****:** Photographs of bacterial strains development. Increasing concentration of solutions A or B, both comprising (R)-5-oxothiomorpholine-3-carboxylic acid (lactam 2), provides a growth advantage to LMD9m in the absence of D-Alanine. Right: Positive control with addition of D-Alanine.
**Figure 3****.** Graph showing SARS-CoV-2 Mpro (2µM) assay overtime with fluorescent substrate in the absence (circles) or presence of (R)-5-oxothiomorpholine-3-carboxylic acid (lactam 2) at 10 µM (squares) or 100 µM (triangles).

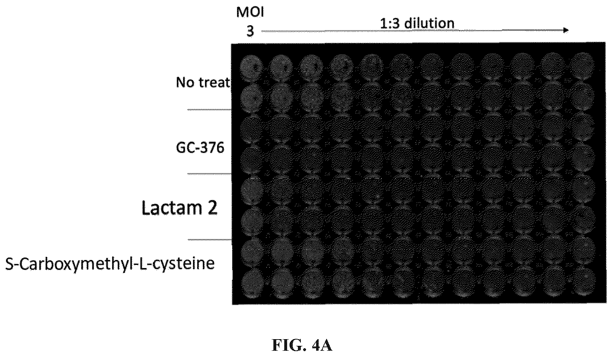

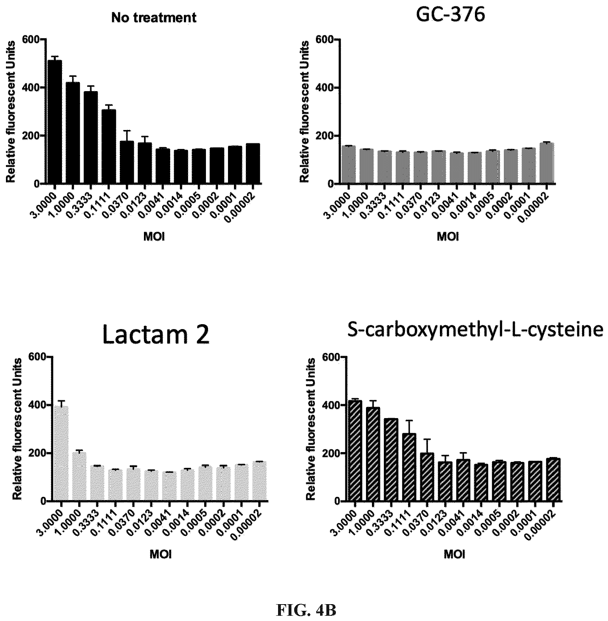

**Figure 4****.** Fig. **4A** represents photograph of SARS-CoV-2 infected Vero cells in absence or in presence of various drugs at 100 µM (GC-376, lactam 2 or S-carboxymethyl-L-cysteine). Light grey color indicates infected cells (fluorescent signal). Fig. **4B** represents graphs showing the quantitative analysis of data represented in Fig. 4A. Fluorescence intensity is representing the amplitude of cell infection by the virus in absence or in presence of various drugs at 100 µM (GC-376, lactam 2 or S-carboxymethyl-L-cysteine). MOI stands for multiplicity of infection (= number of virus/number of cells).

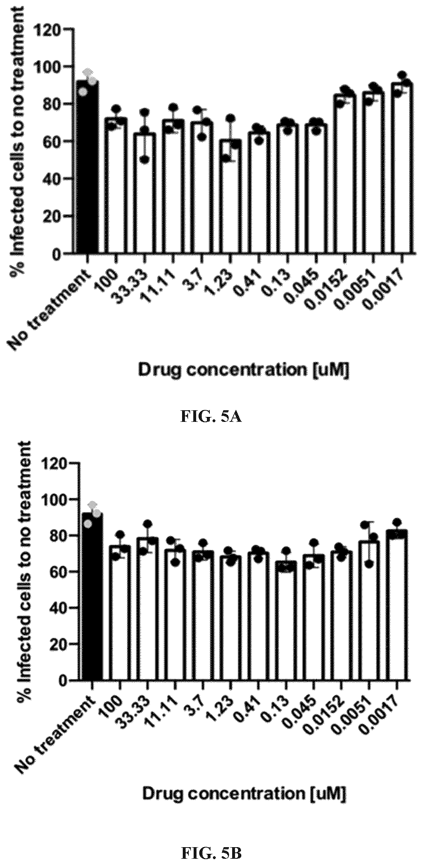

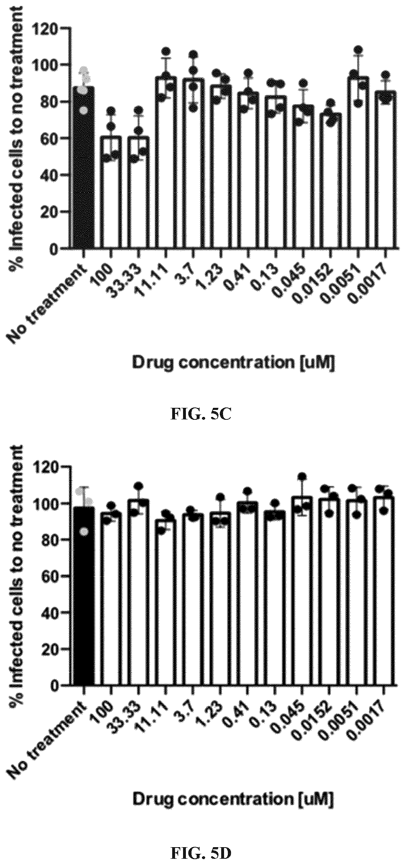

**Figure 5****.** The quantification of SARS-CoV-2 infectivity in Caco-2 cells in the presence of various compounds is evidenced in graphs showing the percentage of infected cells in function of the concentration of tested compound. Fig. **5A****,** lactam 2; Fig. **5B****,** lactam 3, Fig. **5C****,** GC-376; Fig. **5D****,** S-carboxymethyl-L-cysteine.

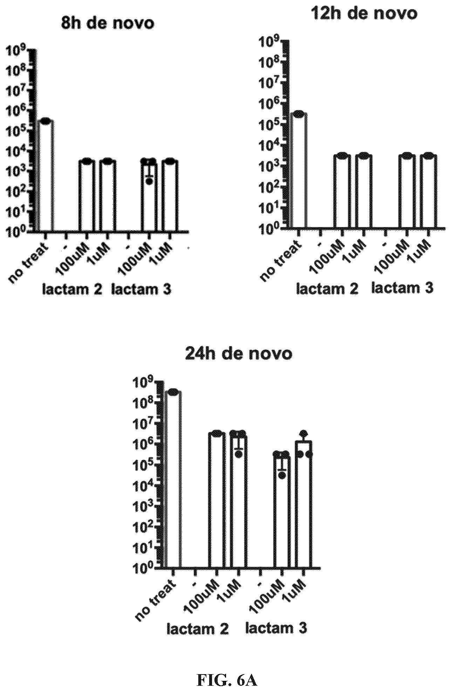

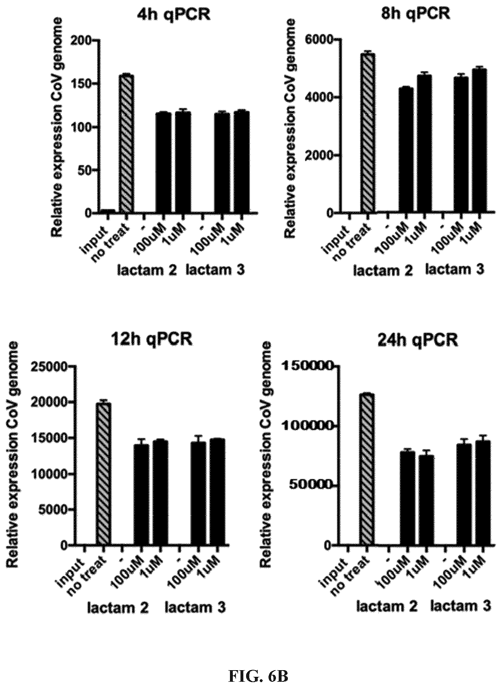

**Figure 6****.** Graphs showing the kinetics of Caco-2 cells infection by SARS-CoV-2 in the presence of various compounds by monitoring overtime the quantity of produced viruses (Fig. **6A**) and the relative quantity of intracellular SARS-CoV-2 RNA (Fig. **6B**).

The viral progeny in supernatants were titrated back on naive Vero cells at 8, 12 and 24h (Fig. **6A**). The intracellular RNA was analyzed by quantitative PCR (qRT-PCR) for virus genome at 4, 8, 12 and 24h (Fig. **6B**).

### EXAMPLES

The present invention is further illustrated by the following examples which do not limit the scope of the invention.

### Example 1: Bacterial reporter strain to assess SARS-CoV-2 Mpro inhibitory effect of tested compounds

***Purpose.*** A bacterial reporter strain was used to demonstrate the inhibitory effect on SARS-CoV-2 Mpro of the compounds of the invention. This strain is a derivative of the Gram-positive *Streptococcus thermophilus* LMD9 strain, called LMD9m (LMD9 *mpro* Δ*alr::alr**). Its chromosome has been engineered to express the SARS-CoV-2 Mpro protease at a permissive tRNAser locus and the endogeneous alanine racemase (Alr) has been engineered by inserting a Mpro cleavage site in a loop exposed at the surface of the enzyme (Alr*).

### Materials and Methods

### Bacterial Strains and Growth Conditions

The bacterial strain used in this study is *Streptococcus thermophilus* LMD-9 wild-type, purchased from ATCC (BAA-491). Growth of the strain was carried out at 37°C in M17 medium (Difco) with added 1% glucose (M17G). The transformations were carried out in skimmed milk medium (SMM, Campina, Belgium). When required, supplements were added to the media at the following concentrations D-alanine (475 µg/ml), chloramphenicol (5 mg/L), erythromycin (2.5 mg/L), 5-fluoroorotic acid (5-FOA) (1.5 mg/ml) (Melford, UK). 5-FOA was diluted in DMSO at a concentration of 100 mg/ml. *S. thermophilus* is an anaerobic bacterium, all growths steps in liquid media are carried out without shaking, and all growth steps in solid media employ anaerobic jars (BBL GasPak Systems; Becton Dickinson, Franklin Lakes, NJ).

The *S. thermophilus* genome sequence can be accessed from GenBank with accession no. CP000419 (LMD-9).

### Construction of the reporter strain

All genetic manipulations were carried out in the chromosome of *S.thermophilus* LMD9 by externally building DNA cassettes by PCR and Gibson assembly and later transforming the strain by inducing natural transformation using a final concentration of 1 µM synthetic XIP peptide (NH2-LPYFAGCL-COOH) ordered at Peptide 2.0 (Chantilly, VA), resuspended at a concentration of 500 µM in 50% DMSO.

The sequence of mpro protease was taken from Kim, D. K., et al. (G3: Genes, Genomes, Genetics, 2020, 10(9), 3399-3402) and was optimized for expression in *S.thermophilus* in this study using the JCat Algorithm (Grote, A., et al., Nucleic Acids Research, 2005, 33(SUPPL. 2), W526-W531) and synthesized from IDT (Integrated DNA Technologies).

The LMD9 mpro Δalr::alr* was constructed by first building the alr* gene (air gene with the mpro recognition site). We introduced a partially degenerate library of 102400 air variants containing the Mpro cleavage site flanked by partially degenerate residues. We selected the alr* variant that is capable of growing on a medium without D-Alanine, indicating that the Mpro cleavage site is tolerated and that the enzyme is active. Consequently, the mpro gene was introduced at the permissive tRNAserine locus to create the LMD9m strain. Protease induced auxotrophy to D-alanine was observed by growing this strain on M17G solid media with and without D-alanine. The mpro gene was then deleted from LMD9m and D-alanine prototrophy was restored.

***Results.*** Figure 1 shows that Mpro is capable of inactivating Alr* and, as a consequence, impair the growth of the strain in a medium without D-Alanine. A compound that is capable of penetrating in the bacteria and inhibit Mpro should provide a growth advantage in a medium without D-Alanine.

### Example 2: Observation of Mpro inhibitory effect of 5-oxothiomorpholine-3-carboxylic acid in bacteria expressing SARS-CoV-2 Mpro

***Purpose.*** The Mpro inhibitory effect of 5-oxothiomorpholine-3-carboxylic acid was assessed in bacterial reporter strain of Example 1.

Two solutions were tested for their capacity to provide a growth advantage to LMD9m in the absence of D-Alanine, in the bacterial strain of Example 1:
- Solution A: S-carboxymethyl-L-cysteine containing less than 1% of (R)-5-oxothiomorpholine-3-carboxylic acid (lactam 2) (0.5 M in S-carboxymethyl-L-cysteine);
- Solution B: mixture of S-carboxymethyl-L-cysteine and (R)-5-oxothiomorpholine-3-carboxylic acid (lactam 2), estimation 70% S-carboxymethyl-L-cysteine and 30% lactam 2 (0.5M in S-carboxymethyl-L-cysteine and lactam 2.

### Materials and Methods

### Preparation of solutions A and B

Chemicals: S-carboxymethyl-L-cysteine (Sigma: Catalogue number C7757-1G) and 1-ethyl-3-(3-dimethylaminopropyl)carbodiimide hydrochloride (EDC-HCl; CarlRoth: Catalogue number 2156.2).

For solution A, 90 mg of S-carboxymethyl-L-Cysteine were dissolved in 500 µl distilled water and 700 µl NaOH 1M per 1.2 ml total volume (final concentration: 75 mg/ml). The pH of the solution was adjusted between 6-7 with 37% Hydrochloric acid. The pH was controlled by adding 10 µl of solution on a pH-indicator strips pH 0-14 universal indicator.

For solution B, 90 mg EDC-HCl was added to a solution A for the cyclisation reaction.

Both solutions were incubated at room temperature on a rotator SB2 Stuart model for 18 hours. The pH of both solutions was evaluated on a test strip directly after the reaction (A: pH = 6-7, B, pH = 7-8). A color change from colorless to yellow was observed in solution B after 18 hours of reaction for the reaction mixture. Both tubes were stored in an ultra-low freezer.

### Mpro inhibition assays

The reporter strain LMD9 mpro Δalr::alr* of Example 1 was inoculated in M17G + D-alanine from a glycerol stock and grown for 3 hours. The cells were washed to remove D-alanine by centrifugation at 2000xg and resuspended in M17G. The washed cells were serially diluted to achieve 10-fold diluted concentrations and later 3 µL of the cells were pipetted onto previously prepared M17G solid plates with different concentration of solutions A and B. The plates were grown anaerobically for 16 hours. The growth of cells in the dilutions at the different concentrations of solutions A and B were compared to serve as a proxy for inhibition of the Mpro.

***Results.*** As shown in Figure 2, both solutions were beneficial for growth. Concentration dependence shows that solution B seems active at a lower concentration (up to 1 nanomolar) than solution A (up to 100 nanomolar), indicating that the (R)-5-oxothiomorpholine-3-carboxylic acid (lactam 2) is acting as the inhibitor.

### Example 3: Inhibition assay with purified SARS-CoV-2 Mpro

***Purpose.*** The Mpro inhibitory effect of the compounds of the invention was also assessed on purified SARS-CoV-2 Mpro.

### Materials and Methods

### Purification of SARS-CoV-2 Mpro

The SARS-CoV-2 Mpro expression plasmid described by Zhang et al. was used (Zhang L, et al., Science, 2020, 368, 409-412). The protease was purified following the protocol of Zhang et al..

### Inhibition assay

Material: (3R)-3-Carboxy-5-oxothiomorpholine, Enamine. LGSAVLQ-Rh110-dP (see structure below) is used as Mpro substrate (from Boston Biochem catalog number S-720).

384-well plate Flat Bottom Black Polystyrol from Greiner.

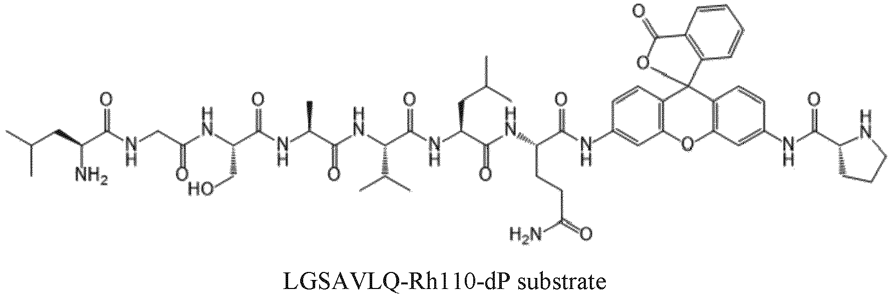

Assays were performed in 50 mM HEPES buffer, pH 7.5, 0.1 mg/ml BSA, 0.01% Triton X-100, and 2 mM DTT. The Mpro enzyme was added at a final concentration of 370 nM in 30µl volume (384-well plate) and mixed with various concentrations of the lactam inhibitor. After 5 min incubation at 25°C, the Mpro fluorogenic substrate is added at a final concentration of 80 µM. Fluorescence is measured immediately after adding the substrate. Fluorescence is followed over time in a TECAN Infinite 200 microplate reader (excitation at 485 nm and emission at 528 nm) at 25°C.

***Results.*** Delta lactam (R)-5-oxothiomorpholine-3-carboxylic acid (lactam 2) was assessed as inhibitor of SARS-CoV-2 Mpro. The activity of Mpro was followed using LGSAVLQ-Rh110-dP fluorogenic substrate. As shown in Figure 3, delta lactam (R)-5-oxothiomorpholine-3-carboxylic acid is an inhibitor of SARS-CoV-2 Mpro. 10 µM of this compound is inhibiting about 50% of SARS-CoV-2 Mpro (at 2µM) while 100 µM of this compound leads to complete inhibition of SARS-CoV-2 Mpro.

### Example 4: Effect of lactam compounds on SARS-CoV2 infection assay in vitro - Vero Cells (monkey kidney)

***Purpose.*** The antiviral effect of the compounds of the invention was assessed by evaluating SARS-CoV2 infectivity in Vero cells.

### Method.

VeroE6 cells (African green monkey kidney epithelial cell line) were from ATCC. All cells below passage 35 were used for experiments. VeroE6 cell culture and virus particle generation and titration was performed as previously described (Stanifer, M. L. et al., Cell Rep. 2020, 32, 107863). Cells were cultured in Dulbecco's modified Eagle medium (DMEM, Life Technologies) containing 10% or 20% fetal bovine serum, respectively, 100 U/mL penicillin, 100 µg/mL streptomycin and 1% non-essential amino acids (complete medium). Cell lines used in these experiments tested negative for mycoplasma using the MycoAlert Plus mycoplasma detection kit (Lonza) as per manufacturer's instructions.

For testing the antiviral activity of selected compounds, VeroE6 cells were seeded one day prior to infection at a density of 3 x 10⁴ cells per well of a clear 96-well plate (Corning). VeroE6 cells (monkey kidney) were pre-treated with 100µM of each tested compound for 1 hour. Virus SARS-CoV-2 at indicated dilutions was added to wells, incubated for 1 hour and was then removed. Tested compounds were added back after virus removal and maintained for the whole infection (incubation at 37°C).

Virus infection was monitored 24 hours post-infection. Plates were fixed with 6% formaldehyde, washed with PBS, and applied to immunostaining using a double-strand RNA-specific antibody (Scicons) suitable to detect SARS-CoV-2 infected cells. Cells were permeabilized with 0.2% Triton-X100 in PBS for 15 min at room temperature, washed once with PBS, and blocked in 2% milk in PBS with 0.02% Tween-20 for 1 hour at room temperature. Cells were incubated with the mouse anti-double strand RNA antibody for 1 hour at room temperature, washed three times with PBS, incubated with the secondary antibody (anti-mouse IgG, conjugated with horseradish peroxidase) for 1 hour at room temperature and subsequently washed four times with PBS. Detection was done by adding TMB (3,3',5,5'-Tetramethylbenzidine) substrate (Thermo Fisher Scientific) and plates were analyzed by photometry at 620 nm using a plate reader. The background absorbance was measured at 450 nm. The percentage of inhibition was determined by dividing the values obtained from the drug-treated cells by the values from the untreated controls.

Tested compounds were:
- (R)-5-oxothiomorpholine-3-carboxylic acid (lactam 2);
- S-carboxymethyl-cysteine, the corresponding linear form of lactam 2; and
- GC-376, which is a known inhibitor of Mpro used in veterinary medicine for treating cats with coronavirus disease.

***Results.*** As shown in Figure 4, (R)-5-oxothiomorpholine-3-carboxylic acid (lactam 2) is reducing the infectivity of SARS-CoV2.

### Example 5: Effect of lactam compounds on SARS-CoV2 infection assay in vitro - on Caco-2 cells (human epithelial)

***Purpose.*** The antiviral effect of the compounds of the invention was assessed by evaluating SARS-CoV2 infectivity in Caco-2 cells.

### Method.

Caco-2 cells were acquired from APC Cork (Alimentary Pharmabiotic Centre, University College Cork, National University of Ireland, Cork, Ireland). Caco-2 cells were propagated in DMEM with GlutaMAX Gibco Cat No 61965-026 and 20% heat inactivated Fetal Bovine Serum (FBS). Cell lines used in these experiments tested negative for mycoplasma using the MycoAlert Plus mycoplasma detection kit (Lonza) as per manufacturer's instructions.

For testing the antiviral activity of selected compounds, Caco-2 cells were seeded one day prior to infection at a density of 3 x 10⁴ cells per well of a clear 96-well plate (Corning). Caco-2 cells (human epithelial) were pre-treated with each tested compound for 1 hour at indicated concentration. Virus SARS-CoV-2 at 0.01 MOI (1 virus for 100 cells) was added to wells, incubated for 1 hour and was then removed. Tested compounds were added back after virus removal and maintained for the whole infection.

Virus infection was quantified 24 hours post-infection, according to the same method as described above in example 4 (Figures 5A-5D).

Results from infecting Caco-2 cells and harvesting intracellular RNA and supernatants at 4, 8, 12 and 24 hours post-infection were also obtained. The cells were treated 1 hour prior to infection with the compounds at 100 µM and 1 µM concentrations. Virus was then added, kept for 1 hour and then removed. The compounds were maintained throughout the time course. The supernatants were titrated back on naive Vero cells (Figure 6A). The RNA was analyzed by quantitative PCR (qRT-PCR) for virus genome (Figure 6B).

Tested compounds were:
- (R)-5-oxothiomorpholine-3-carboxylic acid (lactam 2);
- (S)-6-oxopiperidine-2-carboxylic acid (lactam 3);
- S-carboxymethyl-cysteine, the corresponding linear form of lactam 2; and
- GC-376, which is a known inhibitor of Mpro used in veterinary medicine for treating cats with coronavirus disease.

***Results.*** As shown in Figure 5, lactam 2 is reducing the infectivity of SARS-CoV-2 at concentrations as low as 45 nM (Fig. 5A). As shown in Figure 5B, lactam 3 is also capable of reducing the infectivity of SARS-CoV-2 at concentrations as low as 15 nM.

As show in Figure 6, a reduction of virus production by about 100-fold in the supernatants (after 8, 12 and 24h) (Fig. 6A) and a reduction of about 30% in SARS-Cov2 RNA levels in infected cells was observed (Fig. 6B).

## Claims

1. A compound of formula la, 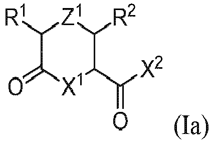 or a pharmaceutically acceptable salt, solvate or prodrug thereof, wherein
X¹ is NH or O;
X² is -OH, -O-alkyl or -NH-R⁵;
Z¹ is S, SO, SO₂, O, CR³ or NR⁴; and
R¹, R², R³ and R⁴ each independently are H, alkyl or aryl; and
R⁵ is H or a peptide group;
for use in the treatment of a disease caused by a viral infection in a subject in need thereof.

2. The compound for use according to claim **1,** wherein the compound is of formula Ia': 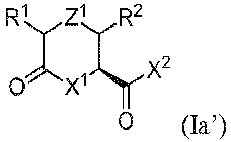 or a pharmaceutically acceptable salt, solvate or prodrug thereof, wherein X¹, X², Z¹, R¹ and R² are as defined in claim 1.

3. The compound for use according to claim 1 or claim 2, wherein the compound is of formula Ia-i or Ia-ii: 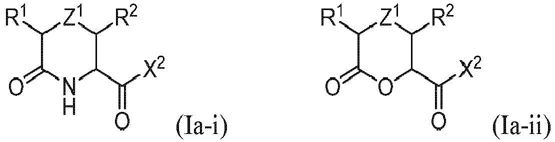 or a pharmaceutically acceptable salt, solvate or prodrug thereof, wherein X², Z¹, R¹ and R² are as defined in claim 1.

4. The compound for use according to any one of claims 1 to 3, wherein the compound is of formula Ia-i' or Ia-ii': 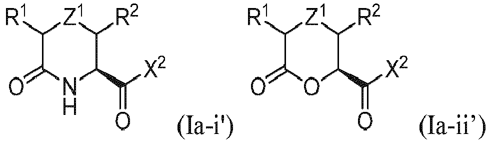 or a pharmaceutically acceptable salt, solvate or prodrug thereof, wherein X², Z¹, R¹ and R² are as defined in claim 1.

5. The compound for use according to any one of claims 1 to 4, wherein the compound is of formula Ia-i-1, Ia-i-2, Ia-i-3, Ia-i-4, Ia-i-5 or Ia-i-6: 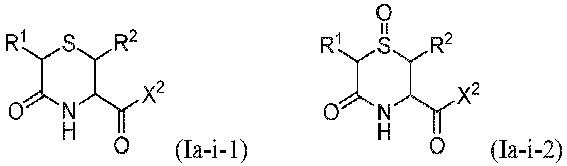 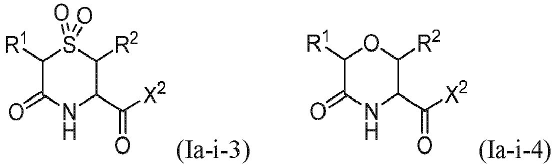 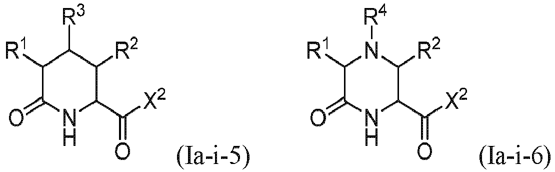 or a pharmaceutically acceptable salt, solvate or prodrug thereof, wherein X², R¹, R², R³ and R⁴ are as defined in claim 1.

6. The compound for use according to any one of claims **1** to **5,** wherein the compound is of formula Ia-i-1', Ia-i-2', Ia-i-3', Ia-i-4', Ia-i-5' or Ia-i-6': 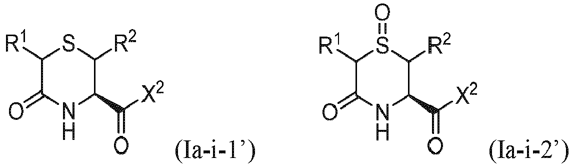 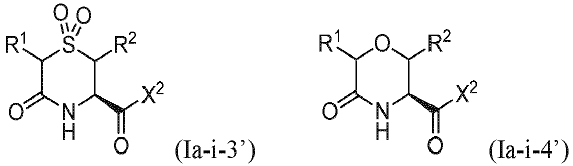 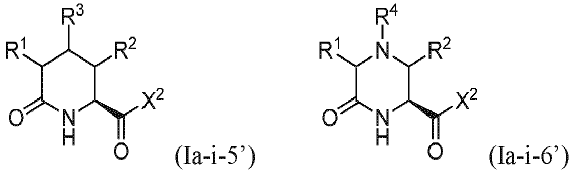 or a pharmaceutically acceptable salt, solvate or prodrug thereof, wherein X², R¹, R², R³ and R⁴ are as defined in claim 1.

7. The compound for use according to any one of claims 1 to 6, wherein the compound is selected from:
(R,S)-5-oxothiomorpholine-3-carboxylic acid;
(R)-5-oxothiomorpholine-3-carboxylic acid;
(R,S)-6-oxopiperidine-2-carboxylic acid;
(S)-6-oxopiperidine-2-carboxylic acid;
(R,S)-6-oxopiperazine-2-carboxylic acid;
(S)-6-oxopiperazine-2-carboxylic acid;
(R,S)-5-oxomorpholine-3-carboxylic acid;
(S)-5-oxomorpholine-3-carboxylic acid;
and pharmaceutically acceptable salts, solvates and prodrugs thereof.

8. The compound for use according to any one of claims **1** to **7,** wherein the viral infection is selected from coronavirus, rhinovirus, respiratory syncytial virus (RSV), seasonal influenza virus, human metapneumovirus, human parainfluenza viruses, highly pathogenic avian influenza A viruses (HPAIV), HIV, Ebola virus, measles virus, and yellow fever virus; preferably the viral infection is a coronavirus infection.

9. The compound for use according to claim **8,** wherein the coronavirus is selected from HCoV-229E, HCoV-NL63, HCoV-OC43, HCoV-HKU1, MERS-CoV, SARS-CoV-1 and SARS-CoV-2; preferably the coronavirus is selected from MERS-CoV, SARS-CoV-1 and SARS-CoV-2; more preferably the coronavirus is SARS-CoV-2.

10. The compound for use according to claim **8** or claim **9,** wherein the coronavirus is SARS-CoV-2 and the disease caused by the coronavirus infection is coronavirus disease 2019 (COVID-19).

11. The compound for use according to any one of claims **1** to **10,** wherein the subject is infected by the virus from less than 10 days, preferably from less than 6 days, more preferably from less than 4 days.

12. The compound for use according to any one of claims **1** to **11,** wherein the compound is to be administered simultaneously, separately or sequentially with at least one further pharmaceutically active agent selected from anti-viral agents, anti-interleukin 6 (anti-IL-6) agents, other agents such as chloroquine, hydroxychloroquine, or dexamethasone, and any mixtures thereof.

13. A method for inhibiting a coronavirus 3C-like protease in a patient in need thereof, comprising administering to said patient an effective amount of a compound of formula Ia as defined in any one of claims **1** to 7.

14. A method for limiting the entry of a coronavirus in patient cells, comprising administering to patient in need thereof an effective amount of a compound of formula Ia as defined in any one of claims **1** to 7.

15. A method for lowering the viral load in a patient infected by a coronavirus, comprising administering to said patient an effective amount of a compound of formula Ia as defined in any one of claims **1** to 7.
